# FASCICULE DE BREVET EUROPEEN

(11) **EP 1 628 696 B1**
(45) Date de publication et mention de la délivrance du brevet: **23.01.2019**
(21) Numéro de dépôt: 04767173.0
(22) Date de dépôt: 25.05.2004
(51) Int. Cl.: A61M 5/50

(54) **SERINGUE PRÉ-REMPLIE AVEC MOYEN DE DÉTECTION DE DÉPLACEMENTS DU PISTON**
VORGEFÜLLTE SPRITZE MIT VORRICHTUNG ZUR DETEKTION EINER BEWEGUNG DES KOLBENS
PRE-FILLED SYRINGE WITH MEANS FOR DETECTING DISPLACEMENT OF THE PISTON

(30) Priorité: 26.05.2003 FR 0306340
(43) Date de publication de la demande: 01.03.2006
(73) Titulaire: Becton Dickinson France, 38800 Le Pont-de-Claix (FR)
(72) Inventeur: PEROT, Frédéric, F-38760 Saint Paul De Varces (FR); FELIX-FAURE, Catherine, F-38000 Grenoble (FR); GRIMARD, Jean-Pierre, F-38450 Vif (FR)
(74) Mandataire: Brédeville, Odile Marie
(86) Numéro de dépôt international: PCT/FR2004/001295
(87) Numéro de publication internationale: WO 2004/105843

(56) Documents cités:
- WO-A-02/15971
- DE-A- 3 107 414
- DE-A- 19 925 621
- US-A- 3 126 004
- US-A- 4 392 852
- US-A- 5 201 721

## Description

La présente invention concerne un dispositif à usage médical, du type seringue pré-remplie, comprenant un moyen de détection de déplacement et/ou de retrait du piston.

Dans le domaine des seringues pré-remplies prêtes à l'emploi, il a déjà été décrit des dispositifs permettant de savoir si la seringue a déjà été utilisée ou non. Ces dispositifs se rapportent généralement à l'extrémité distale de la seringue, c'est-à-dire à l'extrémité de la seringue située au niveau de l'aiguille. Il a ainsi été proposé de recouvrir l'aiguille par un capuchon qu'il faut briser avant de pouvoir utiliser la seringue.

Toutefois, dans le cas des seringues pré-remplies, il peut également être utile de prévoir un dispositif permettant de savoir si le piston de la seringue a été déplacé, voire enlevé, puis réinstallé, afin de déterminer si le liquide médicamenteux présent à l'intérieur du corps de la seringue a pu être contaminé ou même remplacé. Un dispositif selon le préambule de la revendication 1 est par exemple connu de DE 199 25 621 A.

Au cours du processus de fabrication de ce type de seringues, ces dernières sont remplies sous atmosphère stérile, puis ensuite emballées et éventuellement stérilisées. C'est entre ces deux étapes, remplissage et emballage, qu'il convient que la chambre interne de telles seringues ne soit pas exposée ou que le médicament qu'elle contient ne soit pas contaminé ou remplacé.

Le brevet US 3, 126, 004 décrit une seringue comprenant un piston, dont la face proximale, couplée à la tige, est recouverte de cire, cette cire s'étendant jusqu'à la paroi interne de la seringue. Dans ce document, le déplacement du piston brise la cire et l'utilisateur sait ainsi que la seringue a déjà été utilisée. Toutefois, un tel dispositif présente comme inconvénient que lorsque l'utilisateur autorisé veut utiliser la seringue, il doit briser la cire qui se fracture en petits morceaux susceptibles de contaminer l'environnement de l'acte médical.

La présente invention vise à remédier à ce problème en proposant une seringue pré-remplie munie d'un moyen permettant de savoir si le piston de cette seringue a pu déjà être déplacé, voire retiré puis réinstallé, ledit moyen ne présentant aucun risque de contamination lors de l'utilisation par un utilisateur autorisé.

La présente invention porte sur un dispositif à usage médical, du type seringue pré-remplie prête à l'emploi, selon la revendication 1, comprenant un corps tubulaire comprenant à son extrémité distale un nez de réception d'une aiguille et à son extrémité proximale un piston apte à coulisser à l'intérieur dudit corps tubulaire, ledit piston étant disposé dans une position initiale, par rapport au corps tubulaire, définissant un volume utile interne, au moins partiellement rempli par un liquide médicamenteux, le piston étant relié à une tige d'actionnement dudit piston, tel que le corps tubulaire est pourvu, sur sa face interne, d'un moyen de détection de déplacement du piston, ledit moyen étant situé entre la position initiale du piston et l'extrémité proximale du corps tubulaire.

Le moyen de détection de déplacement du piston est situé entre la position initiale du piston, c'est-à-dire après remplissage du dispositif, et l'extrémité proximale du corps tubulaire. Le moyen de détection de déplacement du piston est ainsi distinct du piston. Du fait de sa position sur le corps tubulaire, cette position étant distincte de la position initiale du piston, le moyen de détection de déplacement du piston du dispositif selon l'invention ne présente aucun risque de contamination de l'environnement médical lors de l'utilisation de la seringue par l'utilisateur autorisé. C'est en sollicitant le moyen de détection de déplacement du piston, lors du coulissement du piston, par exemple pour le retirer du corps tubulaire ou pour utiliser la seringue, que le piston sollicite le moyen de détection de déplacement du piston, par exemple en passant sur ce dernier.

Le dispositif selon l'invention permet de savoir facilement si le piston a été retiré puis replacé. En effet, l'utilisateur autorisé est informé de la configuration initiale du moyen de détection de déplacement du piston. Ainsi, lorsqu'il est prêt à utiliser la seringue, deux situations se présentent à lui.

Dans un premier cas, le moyen de détection de déplacement du piston n'a pas été sollicité et il se trouve dans sa configuration ou forme initiale, auquel cas le piston n'a pas été déplacé après le remplissage de la seringue et l'utilisateur peut procéder à l'injection en simplement poussant le piston vers l'extrémité distale du corps tubulaire. Le moyen de détection ou témoin de déplacement du piston étant situé au-dessus de la position initiale du piston, il ne risque donc pas de contaminer le liquide médicamenteux contenu dans le corps tubulaire.

Dans le second cas où le piston a été enlevé puis replacé, le moyen de détection de déplacement du piston a été sollicité : la conséquence peut être que ce moyen a disparu ou qu'il a changé d'aspect, et ce de manière visible ou perceptible pour l'utilisateur. Dans ce cas, l'utilisateur autorisé sait qu'il ne doit pas utiliser la seringue, aucune certitude n'existant sur la véritable nature du liquide médicamenteux contenu dans le corps tubulaire, ce dernier pouvant par exemple avoir été remplacé par un autre liquide ou contaminé frauduleusement par des éléments toxiques ou dangereux ou par l'introduction d'un autre piston.

Dans la présente demande, on entend par extrémité distale d'une pièce l'extrémité la plus éloignée de l'utilisateur du dispositif et par extrémité proximale, l'extrémité la plus proche de l'utilisateur du dispositif.

Dans une forme préférée de réalisation de l'invention, le corps tubulaire est constitué d'un matériau opaque mais translucide. Dans une forme encore plus préférée de réalisation de l'invention, le corps tubulaire est constitué d'un matériau transparent. Dans la présente demande, on entend par matériau transparent un matériau permettant de distinguer les objets à travers son épaisseur. De préférence, le matériau constituant le corps tubulaire est du verre ou du plastique.

Dans le dispositif de l'invention, le moyen de détection de déplacement du piston se présente sous la forme d'une enduction au moins partielle de la face interne du corps tubulaire par un matériau modifiable par cisaillement ou friction. Ainsi, dans une forme de réalisation de l'invention, l'enduction présente une forme initiale, c'est-à-dire après remplissage et avant cisaillement, différente de celle qu'elle présente à l'état cisaillé. Dans la présente invention, en particulier le cisaillement est provoqué par le passage du piston sur le moyen de détection de déplacement du piston. De préférence, ce matériau est un matériau thixotrope. Alors, le matériau constituant l'enduction est à l'état solide ou semi-solide au repos et passe à l'état liquide après cisaillement. Par exemple, ce matériau comprend des microcapsules solides ou semi-solides remplies d'une substance liquide. Ces microcapsules éclatent par cisaillement lors du passage du piston et libèrent leur contenu liquide.

Dans une autre forme de réalisation, le matériau est à l'état visqueux au repos et il est entraîné le long de la face interne du corps tubulaire par le cisaillement provoqué par le passage du piston. De préférence, ce matériau comprend une encre, ou une huile, en particulier de silicone, ou un gel. Dans un tel cas, selon la quantité initiale de matériau présente, des traces de matériau apparaissent sur la face interne du corps tubulaire ou au contraire l'enduction peut être totalement effacée. L'apparition de traces ou la disparition totale de l'enduction sont des témoins de l'utilisation préalable du dispositif et donc de son effraction.

Dans une forme préférée de réalisation de l'invention, cette enduction se présente sous la forme d'un bourrelet annulaire continu, constitué du matériau modifiable par cisaillement.

Dans une forme de réalisation de l'invention, le bourrelet annulaire est discontinu. Par exemple, l'enduction peut comprendre des gouttes de matériau déposées sur la face interne du corps tubulaire dans un plan radial.

Dans une forme préférée de réalisation de l'invention, le moyen de détection de déplacement du piston présente, avant cisaillement, c'est-à-dire dans sa forme non sollicitée, une couleur différente de celle qu'il présente après cisaillement, c'est-à-dire dans sa forme sollicitée. Ainsi, dans la forme de réalisation où l'enduction comprend des microcapsules, ces dernières peuvent contenir un liquide ayant une couleur différente de celle de l'enveloppe extérieure des mocrocapsules. L'apparition de la couleur correspondant à celle du contenu liquide des microcapsules informe directement l'utilisateur que le piston a été déplacé.

Dans une forme de réalisation de l'invention, la tige d'actionnement est munie d'une collerette souple fixée sur ladite tige faisant contact avec la face interne du corps tubulaire et située entre le moyen de détection de déplacement du piston et l'extrémité proximale du corps tubulaire. Grâce à cette collerette, il est possible de déterminer si le piston a été déplacé vers l'extrémité distale du corps tubulaire. En effet, le passage de la collerette sur le moyen de détection de déplacement du piston sollicite ce moyen qui change d'aspect ou disparaît comme expliqué plus haut pour le piston. Ainsi, grâce à cette collerette, tout mouvement du piston, que ce soit vers l'extrémité distale ou au contraire vers l'extrémité proximale du corps tubulaire, peut être détecté par l'utilisateur autorisé.

L'invention porte également sur un procédé de fabrication d'un dispositif à usage médical, du type seringue pré-remplie prête à l'emploi, selon la revendication 12, comprenant un corps tubulaire comprenant à son extrémité distale un nez de réception d'une aiguille, un piston apte à coulisser à l'intérieur du corps tubulaire et un moyen de détection de déplacement du piston, comprenant les étapes successives suivantes :
1°) on ferme l'extrémité distale du corps tubulaire,
2°) on remplit le corps tubulaire d'un liquide médicamenteux jusqu'à une hauteur déterminée du corps tubulaire,
3°) on met en place le piston de façon à enfermer sans air le liquide médicamenteux dans le corps tubulaire, et
4°) on réalise une enduction au moins partielle de la partie de la face interne du corps tubulaire située entre le piston et l'extrémité proximale du corps tubulaire et à distance du piston.

De préférence, après l'étape 4°), on met en place une tige d'actionnement sur le piston en la fixant par vissage ou encliquetage. De préférence, cette tige d'actionnement est munie d'une collerette souple faisant contact avec la face interne du corps tubulaire et située entre l'enduction et l'extrémité proximale du corps tubulaire.

Avantageusement, l'enduction est réalisée à l'aide d'un pinceau, d'une buse ou d'un tampon.

L'invention sera mieux comprise de la description qui suit, en référence au dessin annexé :
- la figure 1 est une vue en coupe d'un dispositif selon l'invention, après remplissage,
- la figure 2 est une vue en coupe d'une variante du dispositif selon l'invention, après remplissage.

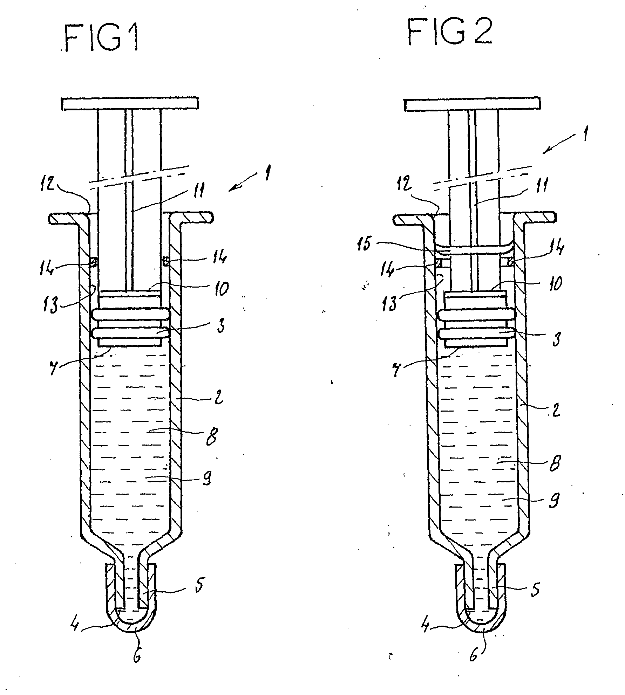

En se référant à la figure 1, est représenté un dispositif 1 comprenant un corps tubulaire 2 en verre et un piston 3 apte à coulisser à l'intérieur du corps tubulaire. Le corps tubulaire 2 comprend à son extrémité distale 4 un nez 5 de réception d'une aiguille creuse (non représentée) fermé par un bouchon 6.

L'extrémité inférieure 7 du piston définit, dans sa position initiale, c'est-à-dire après remplissage, à l'intérieur du corps tubulaire 2, un volume utile interne 8 rempli par un liquide médicamenteux 9. L'extrémité supérieure 10 du piston 3 est reliée à une tige d'actionnement 11 dépassant de l'extrémité proximale 12 du corps tubulaire 2.

Le corps tubulaire 2 est pourvu, sur sa face interne 13, d'un bourrelet annulaire 14 continu, rapporté par enduction, incorporant une encre de couleur, et situé entre la position du piston 3 montrée à la figure 1, c'est-à-dire après remplissage, et l'extrémité proximale 12 du corps tubulaire 2.

Ainsi, si une personne retire ou extrait le piston 3 du corps tubulaire 2 vers son extrémité proximale, après l'étape de remplissage dudit corps, mais avant que le dispositif ne soit emballé, et souille le liquide médicamenteux ou remplace ce liquide par un autre liquide de nature ou de qualité différente, l'encre incorporée dans le bourrelet annulaire sera effacée ou étalée en traces sur la face interne 13 du corps tubulaire 2 lors du passage du piston vers l'extérieur. L'absence d'encre ou la présence de traces ou de bavures par exemple, sera directement observable du fait de la transparence de la paroi du corps tubulaire 2. De cette manière, même si le piston est ensuite replacé à une position identique à sa position initiale au sein du corps tubulaire 2, il est possible de savoir qu'il a été retiré puis réinstallé.

Sur la figure 2 est représenté un dispositif 1 similaire à celui de la figure 1 pour lequel la tige d'actionnement 11 est munie d'une collerette 15 souple, fixée sur la tige 11, faisant contact avec la face interne 13 du corps tubulaire. Les références indiquant les mêmes éléments que dans la figure 1 ont été reprises dans la figure 2. La collerette 15 est située au-dessus du bourrelet annulaire 14. Ainsi, si l'on pousse le piston 3 vers l'extrémité distale 4 du corps tubulaire 2, cette collerette passe sur le bourrelet annulaire 14 et le sollicite en effaçant ou étalant en traces l'encre sur la face interne 13, comme précédemment mais de l'autre côté du bourrelet 14, indiquant ainsi à l'utilisateur autorisé qu'il y a eu déplacement du piston 3 vers l'extrémité distale 4 du corps tubulaire 2.

En référence aux figures 1 et 2, pour réaliser un dispositif 1 selon l'invention, on dispose d'un corps tubulaire 2 comprenant à son extrémité distale 4 un nez 5 de réception d'une aiguille, par exemple une aiguille creuse d'injection hypodermique. On ferme cette extrémité distale 4 par un bouchon 6. On remplit ensuite le corps tubulaire 2, par exemple avec un liquide médicamenteux jusqu'à une hauteur déterminé du corps tubulaire 2, cette hauteur étant par exemple définie par une dose de liquide médicamenteux à injecter. Puis on met en place le piston 3 afin d'enfermer sans air le liquide médicamenteux dans le corps tubulaire 2. On réalise ensuite une enduction au moins partielle de la partie de la face interne du corps tubulaire 2 située entre le piston 3 et l'extrémité proximale 12 du corps tubulaire 2. On peut ensuite rajouter une tige d'actionnement 11 que l'on fixe sur le piston 3 par vissage ou encliquetage. Au préalable, on peut avoir fixé sur cette tige d'actionnement 11 une collerette souple 15 de façon à ce que ladite collerette soit située entre l'enduction et l'extrémité proximale 12 du corps tubulaire 2, une fois la tige d'actionnement fixée sur le piston.

La présente invention est définie par les revendications mais n'est pas limitée aux formes d'exécution décrites dans la présente demande à titre d'exemples.

## Revendications

1. Dispositif (1) à usage médical, du type seringue pré-remplie prête à l'emploi, comprenant un corps tubulaire (2) comprenant à son extrémité distale (4) un nez (5) de réception d'une aiguille et à son extrémité proximale (12) un piston (3) apte à coulisser à l'intérieur dudit corps tubulaire (2), ledit piston (3) étant disposé dans une position initiale, par rapport au corps tubulaire (2), définissant un volume utile interne (8), au moins partiellement rempli par un liquide médicamenteux (9), le piston (3) étant relié à une tige d'actionnement (11) dudit piston, tel que le corps tubulaire (2) est pourvu, sur sa face interne (13), d'un moyen (14) de détection de déplacement du piston (3), ledit moyen (14) étant situé entre la position initiale du piston et l'extrémité proximale (12) du corps tubulaire (2), et étant distinct du piston (3) et étant dans sa configuration initiale à distance du piston, **caractérisé en ce que** ledit moyen (14) de détection se présente sous la forme d'une enduction (14) au moins partielle de la face interne (13) du corps tubulaire (2) par un matériau modifiable par cisaillement ou friction.

2. Dispositif selon la revendication 1, **caractérisé en ce que** le matériau est un matériau thixotrope.

3. Dispositif selon la revendication 1, **caractérisé en ce que** le matériau comprend des microcapsules solides ou semi-solides remplies d'une substance liquide.

4. Dispositif selon la revendication 1, **caractérisé en ce que** le matériau comprend une encre, ou une huile, en particulier de silicone, ou un gel.

5. Dispositif selon l'une quelconque des revendications 1 à 4, **caractérisé en ce que** l'enduction se présente sous la forme d'un bourrelet annulaire (14) continu.

6. Dispositif selon l'une quelconque des revendications 1 à 4, **caractérisé en ce que** l'enduction comprend des gouttes de matériau, déposées sur la face interne (13) du corps tubulaire (2) dans un plan radial.

7. Dispositif selon l'une quelconque des revendications précédentes, **caractérisé en ce que** le corps tubulaire (2) est constitué d'un matériau opaque mais translucide.

8. Dispositif selon l'une quelconque des revendications 1 à 6, caractérisé en que le corps tubulaire (2) est constitué d'un matériau transparent.

9. Dispositif selon l'une des revendications 2 à 8, **caractérisé en ce que** le moyen (14) de détection présente, avant cisaillement, une couleur différente de celle qu'il présente après cisaillement.

10. Dispositif selon l'une quelconque des revendications précédentes, **caractérisé en ce que** la tige d'actionnement (11) est munie d'une collerette souple (15), fixée sur ladite tige (11), faisant contact avec la face interne (13) du corps tubulaire (2) et située entre le moyen (14) de détection de déplacement du piston et l'extrémité proximale (12) du corps tubulaire (2).

11. Dispositif selon l'une quelconque des revendications 1 à 10, caractérisé en que l'enduction présente une forme initiale, c'est-à-dire avant cisaillement, différente de celle qu'elle présente à l'état cisaillé.

12. Procédé de fabrication d'un dispositif (1) à usage médical, du type seringue pré-remplie prête à l'emploi, comprenant un corps tubulaire (2) comprenant à son extrémité distale (4) un nez (5) de réception d'une aiguille, un piston (3) apte à coulisser à l'intérieur du corps tubulaire (2) et un moyen (14) de détection de déplacement du piston (3), comprenant les étapes successives suivantes :
1°) on ferme l'extrémité distale (4) du corps tubulaire (2),
2°) on remplit le corps tubulaire (2) d'un liquide médicamenteux jusqu'à une hauteur déterminée du corps tubulaire (2),
3°) on met en place le piston (3) de façon à enfermer sans air le liquide médicamenteux dans le corps tubulaire (2), et
4°) on réalise une enduction (14) au moins partielle de la partie de la face interne (13) du corps tubulaire (2) située entre le piston et l'extrémité proximale du corps tubulaire (2), à distance du piston, pour former ledit moyen (14) de détection de déplacement du piston (3).

13. Procédé selon la revendication 12, **caractérisé en ce qu'**après l'étape 4°), on met en place une tige d'actionnement (11) sur le piston (3) en la fixant par vissage ou encliquetage.

14. Procédé selon la revendication 13, **caractérisé en ce que** la tige d'actionnement (11) est munie d'une collerette (15) souple faisant contact avec la face interne (13) du corps tubulaire (2) et située entre l'enduction (14) et l'extrémité proximale (12) du corps tubulaire (2).

15. Procédé selon l'une quelconque des revendications 12 à 14, **caractérisé en ce que** l'enduction (14) est réalisée à l'aide d'un pinceau, d'une buse ou d'un tampon.

## Patentansprüche

1. Vorrichtung (1) zur medizinischen Verwendung vom Typ einer gebrauchsfertig vorgefüllten Spritze, umfassend einen rohrförmigen Körper (2), der an seinem distalen Ende (4) einen Ansatz (5) zum Aufnehmen einer Nadel, und an seinem proximalen Ende (12) einen Kolben (3) umfasst, der im Inneren des rohrförmigen Körpers (2) gleiten kann, wobei der Kolben (3) in Bezug auf den rohrförmigen Körper (2) in einer anfänglichen Stellung angeordnet ist, die ein inneres Nutzvolumen (8) definiert, welches mindestens teilweise mit einer medikamentösen Flüssigkeit (9) gefüllt ist, wobei der Kolben (3) mit einem Stab zum Betätigen (11) des Kolbens verbunden ist, derart, dass der rohrförmige Körper (2) an seiner Innenfläche (13) mit einem Mittel (14) zum Erkennen von Bewegung des Kolbens (3) versehen ist, wobei das Mittel (14) zwischen der anfänglichen Stellung des Kolbens und dem proximalen Ende (12) des rohrförmigen Körpers (2) liegt und vom Kolben (3) unabhängig ist und sich in seiner anfänglichen Konfiguration in Abstand zum Kolben befindet, **dadurch gekennzeichnet, dass** sich das Erkennungsmittel (14) in der Form einer mindestens teilweisen Beschichtung (14) der Innenfläche (13) des rohrförmigen Körpers (2) mit einem durch Scherung oder Reibung modifizierbaren Material darstellt.

2. Vorrichtung nach Anspruch 1, **dadurch gekennzeichnet, dass** das Material ein thixotropes Material ist.

3. Vorrichtung nach Anspruch 1, **dadurch gekennzeichnet, dass** das Material feste oder halbfeste Mikrokapseln umfasst, die mit einer flüssigen Substanz gefüllt sind.

4. Vorrichtung nach Anspruch 1, **dadurch gekennzeichnet, dass** das Material eine Tinte, oder ein, insbesondere Silikon-, Öl oder ein Gel umfasst.

5. Vorrichtung nach einem der Ansprüche 1 bis 4, **dadurch gekennzeichnet, dass** sich die Beschichtung in der Form einer durchgehenden ringförmigen Wulst (14) darstellt.

6. Vorrichtung nach einem der Ansprüche 1 bis 4, **dadurch gekennzeichnet, dass** die Beschichtung Materialtropfen umfasst, die in einer radialen Ebene an der Innenfläche (13) des rohrförmigen Körpers (2) abgeschieden sind.

7. Vorrichtung nach einem der vorstehenden Ansprüche, **dadurch gekennzeichnet, dass** der rohrförmige Körper (2) aus einem undurchsichtigen, aber lichtdurchlässigen Material besteht.

8. Vorrichtung nach einem der Ansprüche 1 bis 6, **dadurch gekennzeichnet, dass** der rohrförmige Körper (2) aus einem transparenten Material besteht.

9. Vorrichtung nach einem der Ansprüche 2 bis 8, **dadurch gekennzeichnet, dass** das Erkennungsmittel (14) vor Scherung eine Farbe aufweist, die sich von derjenigen, die sie nach Scherung aufweist, unterscheidet.

10. Vorrichtung nach einem der vorstehenden Ansprüche, **dadurch gekennzeichnet, dass** der Betätigungsstab (11) mit einem am Stab (11) befestigten nachgiebigen Kragen (15) ausgestattet ist, der mit der Innenfläche (13) des rohrförmigen Körpers (2) in Kontakt steht und zwischen dem Mittel (14) zur Erkennung von Bewegung des Kolbens und dem proximalen Ende (12) des rohrförmigen Körpers (2) liegt.

11. Vorrichtung nach einem der Ansprüche 1 bis 10, **dadurch gekennzeichnet, dass** die Beschichtung eine anfängliche Form, das heißt vor Scherung, aufweist, die sich von derjenigen, die sie im gescherten Zustand aufweist, unterscheidet.

12. Verfahren zur Herstellung einer Vorrichtung (1) zur medizinischen Verwendung vom Typ einer gebrauchsfertig vorgefüllten Spritze, die einen rohrförmigen Körper (2), der an seinem distalen Ende (4) einen Ansatz (5) zum Aufnehmen einer Nadel umfasst, einen Kolben (3), der im Inneren des rohrförmigen Körpers (2) gleiten kann, und ein Mittel (14) zum Erkennen von Bewegung des Kolbens (3) umfasst, das die folgenden aufeinanderfolgenden Schritte umfasst:
1) das distale Ende (4) des rohrförmigen Körpers (2) wird verschlossen,
2) der rohrförmige Körper (2) wird bis zu einer bestimmten Höhe des rohrförmigen Körpers (2) mit einer medikamentösen Flüssigkeit gefüllt,
3) der Kolben (3) wird so angebracht, dass die medikamentöse Flüssigkeit ohne Luft im rohrförmigen Körper (2) eingeschlossen wird, und
4) es wird eine mindestens teilweise Beschichtung (14) des Teils der Innenfläche (13) des rohrförmigen Körpers (2), der zwischen dem Kolben und dem proximalen Ende des rohrförmigen Körpers (2) liegt, in Abstand zum Kolben ausgeführt, um das Mittel (14) zum Erkennen von Bewegung des Kolbens (3) zu bilden.

13. Verfahren nach Anspruch 12, **dadurch gekennzeichnet, dass** nach dem Schritt 4) ein Betätigungsstab (11) unter Befestigen desselben durch Verschrauben oder Verrasten am Kolben (3) angebracht wird.

14. Verfahren nach Anspruch 13, **dadurch gekennzeichnet, dass** der Betätigungsstab (11) mit einem nachgiebigen Kragen (15) ausgestattet ist, der mit der Innenfläche (13) des rohrförmigen Körpers (2) in Kontakt steht und zwischen der Beschichtung (14) und dem proximalen Ende (12) des rohrförmigen Körpers (2) liegt.

15. Verfahren nach einem der Ansprüche 12 bis 14, **dadurch gekennzeichnet, dass** die Beschichtung (14) mit Hilfe eines Pinsels, einer Düse oder eines Stempels ausgeführt wird.

## Claims

1. A device (1) for medical use, of the ready-to-use pre-filled syringe type, comprising a tubular body (2) comprising, at its distal end (4), a nose (5) for receiving a needle and, at its proximal end (12), a piston (3) capable of sliding inside said tubular body (2), said piston (3) being disposed in an initial position, relative to the tubular body (2), defining a useful internal volume (8), at least partially filled with a medicinal liquid (9), the piston (3) being connected to an actuating rod (11) of said piston, such that the tubular body (2) is provided, on its inner face (13), with a means (14) for detecting a displacement of the piston (3), said means (14) being located between the initial position of the piston and the proximal end (12) of the tubular body (2), and being distinct from the piston (3) and being in its initial configuration at a distance from the piston,
**characterized in that** said detection means (14) is in the form of an at least partial coating (14) of the inner face (13) of the tubular body (2) by a shear or friction modifiable material.

2. The device according to claim 1, **characterized in that** the material is a thixotropic material.

3. The device according to claim 1, **characterized in that** the material comprises solid or semi-solid microcapsules filled with a liquid substance.

4. The device according to claim 1, **characterized in that** the material comprises an ink, or an oil, in particular silicone oil, or a gel.

5. The device according to any one of claims 1 to 4, **characterized in that** the coating is in the form of a continuous annular bead (14).

6. The device according to any one of claims 1 to 4, **characterized in that** the coating comprises drops of material, deposited on the inner face (13) of the tubular body (2) in a radial plane.

7. The device according to any one of the preceding claims, **characterized in that** the tubular body (2) is constituted of an opaque but translucent material.

8. The device according to any one of claims 1 to 6, **characterized in that** the tubular body (2) is constituted of a transparent material.

9. The device according to any of claims 2 to 8, **characterized in that** the detection means (14) has, before shearing, a different color from the one it has after shearing.

10. The device according to any one of the preceding claims, **characterized in that** the actuating rod (11) is provided with a flexible flange (15), fastened on said rod (11), making contact with the inner face (13) of the tubular body (2) and located between the means (14) for detecting a displacement of the piston and the proximal end (12) of the tubular body (2).

11. The device according to any one of claims 1 to 10, **characterized in that** the coating has an initial shape, that is to say before shearing, different from the one it has in the sheared state.

12. A method for manufacturing a device (1) for medical use, of the ready-to-use pre-filled syringe type, comprising a tubular body (2) comprising, at its distal end (4), a nose (5) for receiving a needle, a piston (3) capable of sliding inside the tubular body (2) and means (14) for detecting a displacement of the piston (3), comprising the following successive steps:
1°) closing the distal end (4) of the tubular body (2),
2°) filling the tubular body (2) with a medicinal liquid, up to a determined height of the tubular body (2),
3°) placing the piston (3) so as to airless enclose the medicinal liquid in the tubular body (2), and
4°) carrying out an at least partial coating (14) of the portion of the inner face (13) of the tubular body (2) located between the piston and the proximal end of the tubular body (2), at a distance from the piston, in order to form said means (14) for detecting a displacement of the piston (3).

13. The method according to claim 12, **characterized in that**, after step 4°), an actuating rod (11) is placed on the piston (3) by fastening it by screwing or snap-fitting.

14. The method according to claim 13, **characterized in that** the actuating rod (11) is provided with a flexible flange (15) making contact with the inner face (13) of the tubular body (2) and located between the coating (14) and the proximal end (12) of the tubular body (2).

15. The method according to any one of claims 12 to 14, **characterized in that** the coating (14) is carried out using a brush, a nozzle or a buffer.
